# EUROPEAN PATENT SPECIFICATION

(11) **EP 4 540 408 B1**
(45) Date of publication and mention of the grant of the patent: **16.09.2026**
(21) Application number: 23733649.0
(22) Date of filing: 12.06.2023
(51) Int. Cl.: C12Q 1/6804, C12Q 1/6806, C12Q 1/6827

(54) **DETECTION OF EPIGENETIC CYTOSINE MODIFICATION**
NACHWEIS EPIGENETISCHER CYTOSINMODIFIKATION
DETECTION DE MODIFICATION EPIGENETIQUE DE CYTOSINE

(30) Priority: 14.06.2022 US 202263366371 P
(43) Date of publication of application: 23.04.2025
(73) Proprietor: F. Hoffmann-La Roche AG, 4070 Basel (CH); Roche Diagnostics GmbH, 68305 Mannheim (DE)
(72) Inventor: HEINDL, Dieter, 82377 Penzberg (DE); BERGMANN, Frank, 82377 Penzberg (DE); CHANG, Shwu shin, Pleasanton, California 94588 (US)
(74) Representative: Hövelmann, Sascha Alexander
(86) International application number: PCT/EP2023/065564
(87) International publication number: WO 2023/242075

(56) References cited:
- WO-A1-2021/161192
- ANONYMOUS: "NEBNext Enzymatic Methyl-seq (EM-seq (TM) )", 1 January 2019 (2019-01-01), XP093084189, Retrieved from the Internet <URL:https://www.neb.com/en/-/media/nebus/files/application-notes/technote_nebnext_enzymatic_methyl-seq.pdf?rev=015e017f782a4bc9b50b61f1b0f3c807&hash=16BD7D037D55735A69E2E66FA792FAE1> [retrieved on 20230921]
- ISSA S ISSA ET AL: "C3 and C6 Modification-Specific OYE Biotransformations of Synthetic Carvones and Sequential BVMO Chemoenzymatic Synthesis of Chiral Caprolactones", CHEMISTRY - A EUROPEAN JOURNAL, JOHN WILEY & SONS, INC, DE, vol. 25, no. 12, 15 January 2019 (2019-01-15), pages 2983 - 2988, XP071848396, ISSN: 0947-6539, DOI: 10.1002/CHEM.201805219
- KUMAR ROY TRIPTESH ET AL: "Ene-Reductase: A Multifaceted Biocatalyst in Organic Synthesis", CHEMISTRY - A EUROPEAN JOURNAL, vol. 28, no. 21, 3 March 2022 (2022-03-03), DE, XP093064509, ISSN: 0947-6539, Retrieved from the Internet <URL:https://onlinelibrary.wiley.com/doi/full-xml/10.1002/chem.202103949> DOI: 10.1002/chem.202103949
- GALARDI FRANCESCA ET AL: "Cell-free DNA-Methylation-Based Methods and Applications in Oncology", 15 December 2020 (2020-12-15), XP093017709, Retrieved from the Internet <URL:https://www.ncbi.nlm.nih.gov/pmc/articles/PMC7765488/pdf/biomolecules-10-01677.pdf> [retrieved on 20230125]
- WANG TONG ET AL: "Enzymatic approaches for profiling cytosine methylation and hydroxymethylation", MOLECULAR METABOLISM, vol. 57, 1 March 2022 (2022-03-01), pages 101314, XP093084218, ISSN: 2212-8778, DOI: 10.1016/j.molmet.2021.101314

## Description

### FIELD OF THE INVENTION

The invention relates to the field of nucleic acid-based diagnostics. More specifically, the invention relates to a method for detecting epigenetic cytosine modifications in nucleic acids, where the epigenetic modifications may have biological and/or clinical significance.

### BACKGROUND OF THE INVENTION

Mapping the epigenetic modifications of DNA and RNA becomes increasingly important as these modifications play a role in several biological processes and diseases including development, aging, cancer, *etc*.). Most relevant to identify epigenetic modifications is detection by sequencing based methods.

Differentiation between cytosine (C) and 5-methylcytosine (5mC) is described by direct sequencing (without pre-amplification) of DNA through a nanopore (e.g., by Pacific Bioscience Single Molecule, Real-Time (SMRT) Sequencing technology) and thereby reading out the different kinetics of nucleotide incorporation opposite C vs. mC by polymerase. Other techniques are using methods to convert C or mC into a T(U) equivalent and subsequent amplification to allow identification when comparing untreated·and converted sample DNA (*see,* Zhao, et al., "Mapping the epigenetic modifications of DNA and RNA," Protein & Cell 11(11):792-808 (2020). The most important methods are: (a) bisulfite sequencing (conversion of C to Uracil (U) by bisulfite treatment), (b) New England Biolab's (NEB's) EM-seq method (oxidation of mC by TET2 enzyme and glucosylation by β-glucosyltransferase (to block enzymatic deaminase reaction) and convert C to U by APOBEC deaminase), (c) TAPS method (TET-assisted pyridine borane sequencing) applying TET enzyme oxidation of mC and subsequent reduction of oxidized mC species by pyridine borane to obtain dihydrocytosine nucleoside which is easily deaminated to give dihydrouracil (a T equivalent), and (d) CLEVER method, which is based on oxidation of mC by TET enzyme and subsequent reaction of 5-formyl-C with malononitrile to give an adduct which acts predominantly as a T equivalent in subsequent PCR amplification (Zhu, et al., "Single-Cell 5-Formylcytosine Landscapes of Mammalian Early Embryos and ESCs at Single-Base Resolution," Cell Stem Cell 20:720-731 (2017)). Instead of enzymatic oxidation of mC by TET enzymes, oxidation can also be performed by chemical means using, *e.g.*, potassium perruthenate (KRuO₄). There are also methods to differentiate the modifications 5-hydroxymethyl-dC (5hmC), 5-formyldC (5fC) or 5-carboxy-dC (5caC) by partly modifying the methods described above.

The TAPS method uses TET enzyme based oxidation of 5-methylcytosine to 5-formylcytosine and/or 5-carboxycytosine and a subsequent reduction of the C5-C6 double bond of the cytosine under deformylation and decarboxylation, respectively. The formed 5,6-dihydrocytosine readily deaminates at the N-4 position to give 5,6-dihydrouracil (DHU). Polymerases read DHU as a T, thus incorporating an A opposite DHU enabling the differentiation of an epigenetic C modification (e.g., mC, hmC,fC or caC) from an unmodified C (*see,* Liu, et al., "Bisulfite-free direct detection of 5-methylcytosine and 5-hydroxymethylcytosine at base resolution," Nat. Biotechnol. 37(4):424-429 (2019)).

The TAPS method described above uses a chemical reduction of the C5-C6 double bond of the cytosine. Boranes (in particular pyridine borane or 2-picoline borane) are employed as a reducing agent. However, these chemical reagents and chemical methods are disadvantageous as they are toxic. Thus, there is a need in the art for a safe and environmentally-friendly method for reducing a C5-C6 double bond of cytosine.

This patent application is directed to the use of enzymatic reduction of the C5-C6 double bond of the cytosine, instead of reduction by chemical means (as in the TAPS method). Thus, chemical reduction by the toxic reagents (in the TAPS method) is replaced by an environmentally-friendly enzymatic reduction process.

### SUMMARY OF THE INVENTION

Thus, there is a need in the art for a safe and environmentally-friendly method for reducing a C5-C6 double bond of cytosine. This patent application is directed to the use of enzymatic reduction of the C5-C6 double bond of the cytosine, instead of reduction by chemical means (as in the TAPS method). Thus, chemical reduction by the toxic reagents (in the TAPS method) is replaced by an environmentally-friendly enzymatic reduction process.

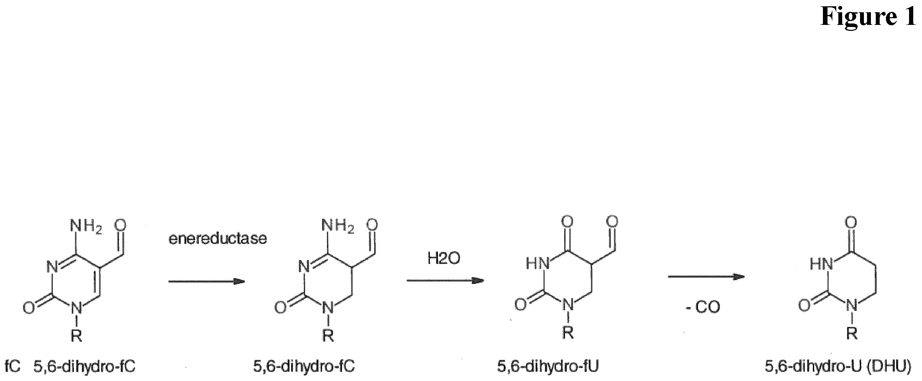

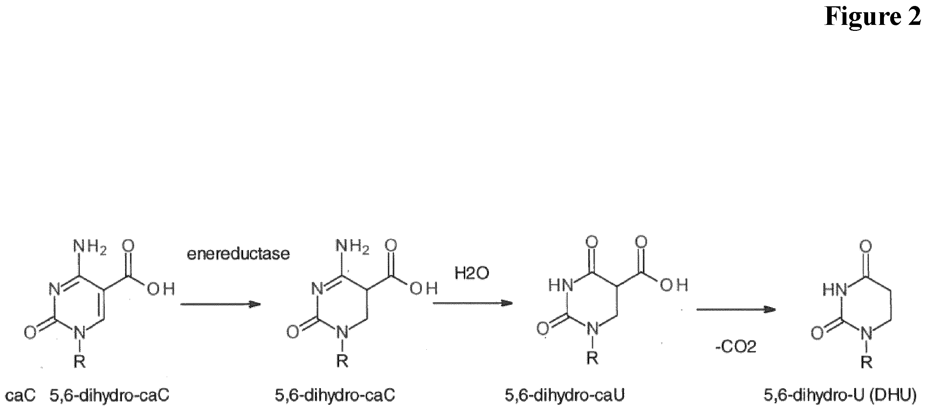

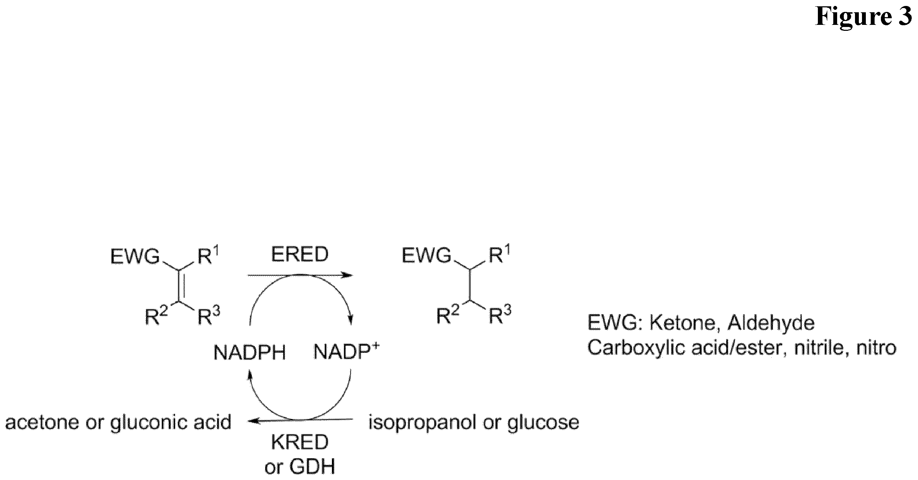

The enzymes employed are enzymes from the group of ene reductases which can reduce electron poor C-C double bonds, in particular double bonds having an electron-withdrawing substituent. In the case of fC or caC, the formyl and the carboxy group are both electron-withdrawing groups, thus being capable of reducing fC and/or caC but not unsubstituted C and U or mC and T. FIG. 1 shows the reduction of 5,6-dihydro-fC (fC) to 5,6-dihydro-U (DHU). FIG. 2 shows the reduction of 5,6-dihydro-caC (caC) to 5,6-dihydro-U (DHU). Ene reductases (ERED) can be purchased commercially (e.g., Codexis, Redwood City, CA, US). FIG. 3 shows the reaction of interest, wherein an ene reductase (ERED) reduces a double bond (taken from CODEXIS, Codex ERED Screening Kit, Screening Protocol, document #PRO-004-003, page 1 (accessed June 10, 2022, at https://www.codexis-estore.com/ files/ugd/5a7b2a 5fbf41f3ae2741a081894f64424552d1.pdf).

One embodiment is directed to a method for reducing a C5-C6 double bond of cytosine in a nucleic acid, wherein the method comprises contacting cytosine in the nucleic acid with an ene reductase. In one embodiment, the cytosine is a 5-Formylcytosine (5fC). In one embodiment, the cytosine is a 5-Carboxylcytosine (5caC). In one embodiment, the cytosine is a 5,6-dihydro-fC. In one embodiment, the cytosine is a 5,6-dihydro-caC. In one embodiment, the cytosine is a 2'-Deoxy-5-formylcytidine. In one embodiment, the cytosine is a 2'-Deoxy-5-carboxycytidine. In one embodiment, the cytosine is a 5-Formyl-2'-deoxycytidine. In one embodiment, the cytosine is 5-Formyl-dC. In one embodiment, the cytosine is a 5-Carboxy-2'-deoxycytidine. In one embodiment, the cytosine is a 5-Carboxy-dC. In one embodiment, contacting the cytosine in the nucleic acid with an ene reductase reduces the cytosine to 5,6-dihydro-U (DHU). In one embodiment, the DHU is subsequently converted to a Thymine. In one embodiment, reduction of the C5-C6 double bond of cytosine in a nucleic acid by contacting the cytosine in the nucleic acid with an ene reductase is a part of a method for detecting an epigenetic cytosine modification.

Another embodiment is directed to a method for detecting an epigenetic cytosine modification, wherein the method comprises at least the step of reducing a C5-C6 double bond of cytosine in a nucleic acid by contacting cytosine in the nucleic acid with an ene reductase. In one embodiment, the cytosine is a 5-Formylcytosine (5fC). In one embodiment, the cytosine is a 5-Carboxylcytosine (5caC). In one embodiment, the cytosine is a 5,6-dihydro-fC. In one embodiment, the cytosine is a 5,6-dihydro-caC. In one embodiment, the cytosine is a 2'-Deoxy-5-formylcytidine. In one embodiment, the cytosine is a 2'-Deoxy-5-carboxycytidine. In one embodiment, the cytosine is a 5-Formyl-2'-deoxycytidine. In one embodiment, the cytosine is 5-Formyl-dC. In one embodiment, the cytosine is a 5-Carboxy-2'-deoxycytidine. In one embodiment, the cytosine is a 5-Carboxy-dC. In one embodiment, contacting the cytosine in the nucleic acid with an ene reductase reduces the cytosine to 5,6-dihydro-U (DHU). In one embodiment, the DHU is subsequently converted to a thymine.

Unless otherwise defined, all technical and scientific terms used herein have the same meaning as commonly understood by one of ordinary skill in the art to which this invention belongs. Although methods and materials similar or equivalent to those described herein can be used in the practice or testing of the present subject matter, suitable methods and materials are described below. In addition, the materials, methods, and examples are illustrative only and not intended to be limiting. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

### BRIEF DESCRIPTION OF THE FIGURES

The patent or application file contains at least one drawing executed in color. Copies of this patent or patent application publication with color drawing(s) will be provided by the Office upon request and payment of the necessary fee.
FIG. 1 shows the reduction of 5,6-dihydro-fC (fC) to 5,6-dihydro-U (DHU).
FIG. 2 shows the reduction of 5,6-dihydro-caC (caC) to 5,6-dihydro-U (DHU).
FIG. 3 shows the reaction of interest, wherein an ene reductase (ERED) reduces a double bond (taken from CODEXIS, Codex ERED Screening Kit, Screening Protocol, document #PRO-004-003, page 1 (accessed June 10, 2022, at https://www.codexis-estore.com/ files/ugd/Sa7b2a_5fbf41f3ae2741a081894f64424552d1.pdf).

### DETAILED DESCRIPTION OF THE INVENTION

### Abbreviations

Some abbreviations used throughout this disclosure are listed below.
C - cytosine
T - thymine
U - uracil
DHU - dihydrouracil
5mC - 5-methylcytosine
5hmC - 5-hyrdoxymethyl cytosine
5ghmC - 5-glucosyl-hydroxymethyl cytosine
5fC - 5-formylcytosine
5caC - 5-carboxycytosine
TET - ten-eleven translocation dioxygenase
TAPS - TET-assisted pic-borane sequencing
CAPS - chemically-assisted pic-borane sequencing
oxBS or oxBS-Seq - oxidative bisulfite sequencing

5-Methyl cytosine and 5-hydroxymethyl cytosine (5mC and 5hmC) are important epigenetic biomarkers with many clinical applications in oncology, prenatal testing and other fields. Until recently, base-level detection of methylation was achieved by reacting unmethylated cytosines with bisulfite followed by PCR, array hybridization or sequencing. Unmethylated cytosines (C) would read as thymine (T) after reacting with bisulfite, while methylated cytosines (5mC and 5hmC) would read as C. Unfortunately, bisulfite treatment leads to degradation of large portion of sample nucleic acid making it unsuitable for applications requiring high sensitivity. For example, the method is unsuitable for latest applications analyzing cell-free nucleic acid such as cell-free DNA.

Recently, less harsh methods for the detection of methylated cytosines have been disclosed. The newest methods involve modification of the methylated cytosines instead of the unmethylated cytosines, as is the case with bisulfite treatment (Liu, et al., Nature Biotechnology 37:424-429 (2019) (hereinafter, "Liu, *et al.* (2019)")). The stepwise oxidation of methyl-cytosines (5mC) via 5-hydroxymethyl cytosine (5hmC) to formyl cytosine (5fC) and carboxyl cytosine (5caC) is performed using ten-eleven translocation dioxygenases (TET) in the presence of Fe(II) ions and alpha-ketoglutarate.

Liu, *et al.* (2019) further described reducing 5fC (and 5caC) using borane derivatives (such a pyridine borane, picoline borane and others) to dihydrouracil (DHU). DHU is then read by uracil-tolerant nucleic acid polymerases as T in subsequent amplification and sequencing. As a result, methylated C is read as T, while unmethylated C is unchanged. This TET and picoline-borane based method called TAPS (TET-assisted picoline-borane sequencing) does not cause DNA degradation as much as bisulfite treatment and allows detection of the signal directly instead of subtracting background to obtain signal. Both advantages would allow higher alignment rates, possibly lower sequencing depth and recover higher molecular diversity from the sample.

Another technique termed CAPS (Chemically Assisted Picoline-borane Sequencing) involves the selective conversion of 5hmC to 5fC using potassium perruthenate (KRuO₄). The use of KRuO₄ as a chemical alternative to TET is known from a technique termed Oxidative Bisulfite Sequencing or oxBS-seq (*see,* Booth, et al., Science 336(6083):934-937 (2012) (hereinafter, "Booth, *et al.* (2012)")). The 5fC obtained by potassium perruthenate conversion becomes a favorable target for further processing by, *e.g.,* borane treatment or any other downstream method.

Yet another sequencing technique is an alternative to the reduction of 5fC with borane. This method involves forming an adduct of 5fC recognized as T. The adduct is formed with the use of malononitrile (*see,* Zhu, et al. Cell Stem Cell 20:720-731 (2017) (hereinafter, "Zhu, *et al.* (2017)")). TAPS, CAPS and the malononitrile method of Zhu, *et al.* (2017) are superior to bisulfite method in that they avoid the harsh chemical treatment and the resulting loss of sample nucleic acids.

However, these methods have a disadvantage of often using a chemical reduction of the C5-C6 double bond of the cytosine. Boranes (in particular pyridine borane or 2-picoline borane) are employed as a reducing agent. However, these chemical reagents and chemical methods are disadvantageous as they are toxic. Thus, there is a need in the art for a safe and environmentally-friendly method for reducing a C5-C6 double bond of cytosine.

This patent application is directed to the use of enzymatic reduction of the C5-C6 double bond of the cytosine, instead of reduction by chemical means (as in the TAPS method). Thus, chemical reduction by the toxic reagents (in the TAPS method) is replaced by an environmentally-friendly enzymatic reduction process.

In some embodiments, the invention is a method of detecting an epigenetic modification, specifically, an epigenetic cytosine modification in nucleic acids (including, but not limited to, cytosine methylation). The state of the art methods for detecting an epigenetic cytosine modification can include reducing a C5-C6 double bond using harsh chemical methods. However, this patent application offers an improvement in the art. This patent application is directed to the use of enzymatic reduction (*i.e.,* not chemical reduction) of the C5-C6 double bond of cytosine.

The present invention involves a method of manipulating nucleic acids from a sample. In some embodiments, the sample is derived from a subject or a patient. In some embodiments the sample may comprise a fragment of a solid tissue or a solid tumor derived from the subject or the patient, *e.g.,* by biopsy. The sample may also comprise body fluids that may contain nucleic acids (e.g., urine, sputum, serum, blood or blood fractions, *i.e.,* plasma, lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, or fecal samples). In other embodiments, the sample is a cultured sample, *e.g.,* a tissue culture containing cells and fluids from which nucleic acids may be isolated. In some embodiments, the nucleic acids of interest in the sample come from infectious agents such as viruses, bacteria, protozoa or fungi.

The present invention involves manipulating isolated nucleic acids isolated or extracted from a sample. Methods of nucleic acid extraction are well known in the art (see, Sambrook et al., "Molecular Cloning: A Laboratory Manual," 1989, 2nd Ed., Cold Spring Harbor Laboratory Press: New York, N.Y.). A variety of kits are commercially available for extracting nucleic acids (DNA or RNA) from biological samples (e.g., KAPA Express Extract (Roche Sequencing Solutions, Pleasanton, Cal.) and other similar products from BD Biosciences Clontech (Palo Alto, Cal.), Epicentre Technologies (Madison, Wisc.); Gentra Systems, (Minneapolis, Minn.); and Qiagen (Valencia, Cal.), Ambion (Austin, Tex.); BioRad Laboratories (Hercules, Cal.); and more.

In some embodiments, nucleic acids are extracted, separated by size and optionally, concentrated by epitachophoresis as described, e.g., in International Patent Publication Nos. WO2019/092269 and WO2020/074742.

The present invention involves detecting an epigenetic modification, specifically, an epigenetic cytosine modification in nucleic acids (including, but not limited to, cytosine methylation). The nucleic acid sequences that are subject to conditional epigenetic modification are the target sequences analyzed by the method disclosed herein. The same nucleic acid sequence may or may not have the epigenetic modification characterized by methylation of cytosines at the 5-position (5mC or 5hmC). In some embodiments, a set or a panel of target nucleic acids are probed for the presence of methylation. For example, as shown in Patai, et al. "Comprehensive DNA Methylation Analysis Reveals a Common Ten-Gene Methylation Signature in Colorectal Adenomas and Carcinomas" PLOS ONE 10(8): e0133836 (2015), and in Onwuka, et al., "A panel of DNA methylation signature from peripheral blood may predict colorectal cancer susceptibility," BMC Cancer 20, 692 (2020), methylation of biomarkers in a panel of methylation biomarkers is indicative of the presence of colorectal cancer in the patient. Accordingly, testing any known or future panels of methylation biomarkers for prognostic or diagnostic purposes is envisioned with the method disclosed herein.

In some embodiments, the entire genome of an organism is probed for the presence of methylation. The method of the instant invention includes detecting methylation in all sites throughout the genome of an organism to diagnose a disease or condition or predisposition to a disease or condition using the sequence analysis and artificial intelligence tools described, *e.g.,* in Shull, et al. "Sequencing the cancer methylome," Methods Mol Biol. 1238:627-635 (2015).

n some embodiments, the invention comprises improved steps of detecting methylated cytosine in nucleic acids by forming and detecting 5-carboxylcytosine (5caC) and 5-formylcytosine (5fC), wherein 5fC, 5caC or a mixture of 5fC and 5caC are formed by one of the methods described herein above. The method involves contacting a sample containing a nucleic acid comprising 5fC and/or 5caC with an enzyme, particularly, an ene reductase t to form dihydrouracil (DHU).

Following the formation of DHU, the nucleic acid with DHU is subjected to sequencing. In some embodiments, sequencing is by a next-generation massively parallel sequencing process. Sequencing results in a test sequence wherein the DHU are read as thymine (T), *i.e.,* the sequencing polymerase is able to accommodate the adduct or DHU in the strand being copied, and to incorporate an adenine (A) opposite the DHU. The method further comprises a step of comparing the test sequence with a reference sequence, wherein a change from a cytosine (C) in the reference sequence to a thymine (T) in the corresponding position in the test sequence indicates the presence of methylated cytosine in the test nucleic acid.

In some embodiments, the nucleic acids in the sample are amplified prior to sequencing. In some embodiments, amplification utilizes a B-family polymerase efficiently incorporating an adenine (A) nucleotide opposite the DHU. In this embodiment, the sequencing may proceed with any polymerase suitable for the sequencing process as the DHU have already been recognized as T by the amplification polymerase.

In some embodiments, the nucleic acid in the sample is ligated to adaptors, wherein adaptors comprise elements useful in amplification and sequencing. An adaptor comprises at least one of the following: barcode, primer binding site and ligation site.

In some embodiments, the invention is an improved method of detecting a methylated cytosine nucleotide in a nucleic acid, the method comprising: (i) ligating adaptors to a nucleic acid in a sample wherein adaptors comprise amplification primer binding sites; (ii) forming a reaction mixture by contacting the sample containing adaptor-ligated nucleic acid with TET capable of converting the methylated cytosine in the nucleic acid into 5-carboxycytosine (5caC) or a mixture of 5-formyl cytosine (5fC) and 5caC; (iii) contacting the reaction mixture with an enzyme capable of reacting with 5fC and 5caC in the nucleic acid to form DHU (including, but not limited to, ene reductases) (iv) incubating the reaction mixture for no more than about 1 hour wherein at least 90% of 5fC and 5caC has formed DHU; (v) amplifying the adapted nucleic acids utilizing a DNA polymerase and primers capable of binding to the primer-binding sites, wherein the DNA polymerase reads DHU as thymine (T) during amplification; (vi) sequencing the amplified nucleic acid to obtain a test sequence; (vii) comparing the test sequence with a reference sequence, wherein a change from a cytosine (C) in the reference sequence to a thymine (T) in the corresponding position in the test sequence indicates the presence of 5mC in the nucleic acid. In some embodiments, in steps (iii) and (iv) the borane derivative is present in a non-aqueous solvent, *e.g.*, ethanol or methanol. In some embodiments, in steps (iii) and (iv) the borane derivative is present in a solution comprising an organic acid such acetic acid.

In some embodiments, the invention utilizes an adaptor added to one or both ends of a nucleic acid or nucleic acid strand. Adaptors of various shapes and functions are known in the art (*see, e.g.,* International Patent Application No. PCT/EP2019/05515 filed on February 28, 2019, U.S. Patent Nos. 8,822,150 and 8,455,193). In some embodiments, the function of an adaptor is to introduce desired elements into a nucleic acid. The adaptor-borne elements include at least one of nucleic acid barcode, primer binding site or a ligation-enabling site.

The adaptor may be double-stranded, partially single stranded or single stranded. In some embodiments, a Y-shaped, a hairpin adaptor or a stem-loop adaptor is used wherein the double-stranded portion of the adaptor is ligated to the double stranded nucleic acid formed as described herein.

In some embodiments, the adaptor molecules are *in vitro* synthesized artificial sequences. In other embodiments, the adaptor molecules are *in vitro* synthesized naturally-occurring sequences. In yet other embodiments, the adaptor molecules are isolated naturally occurring molecules or isolated non naturally-occurring molecules.

The double-stranded or partially double-stranded adaptor oligonucleotide can have overhangs or blunt ends. In some embodiments, the double-stranded DNA may comprise blunt ends to which a blunt-end ligation can be applied to ligate a bluntended adaptor. In other embodiments, the blunt ended DNA undergoes A-tailing where a single A nucleotide is added to the blunt ends to match an adaptor designed to have a single T nucleotide extending from the blunt end to facilitate ligation between the DNA and the adaptor. Commercially available kits for performing adaptor ligation include AVENIO ctDNA Library Prep Kit or KAPA HyperPrep and HyperPlus kits (Roche Sequencing Solutions, Pleasanton, CA). In some embodiments, the adaptor ligated (adapted) DNA may be separated from excess adaptors and unligated DNA.

In some embodiments, the invention includes the use of a barcode. In some embodiments, the method of detecting epigenetic modifications includes sequencing. The nucleic acid processed as described herein is subjected to sequencing; preferably, massively parallel single molecule sequencing. Analyzing individual molecules by massively parallel sequencing typically requires a separate level of barcoding for sample identification and error correction. The use of molecular barcodes such as described in U.S. Patent Nos. 7,393,665, 8,168,385, 8,481,292, 8,685,678, and 8,722,368. A unique molecular barcode is added to each molecule to be sequenced to mark molecule and its progeny (e.g., the original molecule and its amplicons generated by PCR). The unique molecular barcode (UID) has multiple uses including counting the number of original target molecules in the sample and error correction (Newman, et al., "An ultrasensitive method for quantitating circulating tumor DNA with broad patient coverage," Nature Medicine doi:10.1038/nm.3519 (2014)).

In some embodiments, unique molecular barcodes (UIDs) are used for sequencing error correction. The entire progeny of a single target molecule is marked with the same barcode and forms a barcoded family. A variation in the sequence not shared by all members of the barcoded family is discarded as an artefact. Barcodes can also be used for positional deduplication and target quantification, as the entire family represents a single molecule in the original sample (Newman, et al. "Integrated digital error suppression for improved detection of circulating tumor DNA," Nature Biotechnology 34:547 (2016)).

In some embodiments of the invention, the adaptor ligated to one or both ends of the barcoded target nucleic acid comprises one or more barcodes used in sequencing. A barcode can be a UID or a multiplex sample ID (MID or SID) used to identify the source of the sample where samples are mixed (multiplexed). The barcode may also be a combination of a UID and an MID. In some embodiments, a single barcode is used as both UID and MID. In some embodiments, each barcode comprises a predefined sequence. In other embodiments, the barcode comprises a random sequence. In some embodiments of the invention, the barcodes are between about 4-20 bases long so that between 96 and 384 different adaptors, each with a different pair of identical barcodes are added to a human genomic sample. In some embodiments, the number of UIDs in the reaction can be in excess of the number of molecules to be labelled. A person of ordinary skill would recognize that the number of barcodes depends on the complexity of the sample (i.e., expected number of unique target molecules) and would be able to create a suitable number of barcodes for each experiment.

In some embodiments, the method involves forming a library comprising nucleic acids from a sample. The library consists of a plurality of nucleic acids ready for sequencing or another type of detection method, *e.g.,* PCR. A library can be stored and used multiple times for further processing such as amplification or sequencing of the nucleic acids in the library. In some embodiments, the library is the input nucleic acid in which methylation is detected by the method described herein. In other embodiments, the library is formed from nucleic acids that have undergone the methylation detection reactions described herein.

In some embodiments, the nucleic acids processed for detection of epigenetic modifications according to the method described herein are sequenced. Any of a number of sequencing technologies or sequencing assays can be utilized. The term "Next Generation Sequencing (NGS)" as used herein refers to sequencing methods that allow for massively parallel sequencing of clonally amplified molecules and of single nucleic acid molecules.

Non-limiting examples of sequence assays that are suitable for use with the methods disclosed herein include nanopore sequencing (U.S. Patent Publication Nos. US2013/0244340, US2013/0264207, US2014/0134616, US2015/0119259 and US2015/0337366), Sanger sequencing, capillary array sequencing, thermal cycle sequencing (Sears, et al., Biotechniques, 13:626-633 (1992)), solid-phase sequencing (Zimmerman, et al., Methods Mol. Cell Biol., 3:39-42 (1992)), sequencing with mass spectrometry such as matrix-assisted laser desorption/ionization time-of-flight mass spectrometry (MALDI-TOF/MS; Fu, et al., Nature Biotech., 16:381-384 (1998)), sequencing by hybridization (Drmanac et al., Nature Biotech., 16:54-58 (1998), and NGS methods, including but not limited to sequencing by synthesis (e.g., HiSeq^{™}, MiSeq^{™}, or Genome Analyzer, each available from Illumina), sequencing by ligation (e.g., SOLiD^{™}, Life Technologies), ion semiconductor sequencing (e.g., Ion Torrent^{™}, Life Technologies), and SMRT^{®} sequencing (e.g., Pacific Biosciences).

Commercially available sequencing technologies include: sequencing-by-hybridization platforms from Affymetrix Inc. (Sunnyvale, Calif.), sequencing-by-synthesis platforms from Illumina/Solexa (San Diego, Calif.) and Helicos Biosciences (Cambridge, Mass.), sequencing-by-ligation platform from Applied Biosystems (Foster City, Calif.). Other sequencing technologies include, but are not limited to, the Ion Torrent technology (ThermoFisher Scientific), and nanopore sequencing (Genia Technology from Roche Sequencing Solutions, Santa Clara, Cal.), and Oxford Nanopore Technologies (Oxford, UK).

In some embodiments, the sequencing step involves sequence aligning. In some embodiments, aligning is used to determine a consensus sequence from a plurality of sequences, e.g., a plurality having the same unique molecular ID (UID). The molecular ID is a barcode that can be added to each molecule prior to sequencing or if amplification step is included, prior to the amplification step. In some embodiments, a UID is present in the 5'-portion of the RT primer. Similarly, a UID can be present in the 5'-end of the last barcode subunit to be added to the compound barcode. In other embodiments, a UID is present in an adaptor and is added to one or both ends of the target nucleic acid by ligation.

In some embodiments, a consensus sequence is determined from a plurality of sequences all having an identical UID. The sequenced having an identical UID are presumed to derive from the same original molecule through amplification. In other embodiments, UID is used to eliminate artifacts, i.e., variations existing in the progeny of a single molecule (characterized by a particular UID). Such artifacts resulting from PCR errors or sequencing errors can be eliminated using UIDs.

In some embodiments, the number of each sequence in the sample can be quantified by quantifying relative numbers of sequences with each UID among the population having the same multiplex sample ID (MID). Each UID represents a single molecule in the original sample and counting different UIDs associated with each sequence variant can determine the fraction of each sequence variant in the original sample, where all molecules share the same MID. A person skilled in the art will be able to determine the number of sequence reads necessary to determine a consensus sequence. In some embodiments, the relevant number is reads per UID ("sequence depth") necessary for an accurate quantitative result. In some embodiments, the desired depth is 5-50 reads per UID.

In some embodiments, the invention is a kit including components and tools for performing an improved method of detecting DNA methylation described herein. In some embodiments, the kit includes components for detecting cytosine methylation in nucleic acids by detecting a product of in vitro oxidized 5-methyl cytosine (5mC) or 5-hyrdoxymethyl cytosine (5hmC). In some embodiments, the product is 5-formyl cytosine (5fC) or 5-carboxy cytosine (5caC). In other embodiments, the kit further includes components for performing in vitro oxidation of 5-methyl cytosine (5mC) or 5-hyrdoxymethyl cytosine (5hmC) to 5-formyl cytosine (5fC) or 5-carboxy cytosine (5caC).

In some embodiments, the kit includes an enzyme. In some embodiments, the enzyme is an ene reductase. In some embodiments, the kit further includes an organic acid. In some embodiments, the kid includes instructions on using the organic acid (such as acetic acid) in a method of detecting DNA methylation including borane derivatives in a non-aqueous solvent as described herein. In some embodiments, the kit further includes a buffer such as MES or TRIS.

In some embodiments, the kit includes malononitrile and a non-aqueous solvent. The non-aqueous solvent is selected from ethanol and methanol. In other embodiments, instead of including the non-aqueous solvent, the kit includes instructions on using the non-aqueous solvent (such as ethanol or methanol) in a method of detecting DNA methylation with malononitrile as described herein. In some embodiments, the kit further comprises an organic acid and a primary, a secondary or a tertiary amine. The organic acid may be acetic acid and the amine may be triethanolanime. In other embodiments, the kit includes instructions on using the organic acid and the amine (such as acetic acid and triethanolamine) in a method of detecting DNA methylation with malononitrile as described herein. In some embodiments, the kit further includes a buffer such as MES or TRIS.

In some embodiments, the kit further includes TET enzyme for *in vitro* oxidation of 5-methyl cytosine (5mC) or 5-hyrdoxymethyl cytosine (5hmC) to 5-carboxy cytosine (5caC). In some embodiments, TET is selected from mouse TET1, TET2 or TET3 (mTET1, 2 or 3), human TET1, TET2 or TET3 (hTET1, 2 or 3), Naegleria TET (NgTET), Coprinopsis cinerea (CcTET). In some embodiments, TET is Naegleria TET-like oxygenase (NgTET1). In some embodiments, TET is a wildtype protein. In other embodiments, TET is a mutant protein. In some embodiments, the kit further includes one or more co-factors selected from alpha-ketoglutarate and a source of Fe(II) ions.

In some embodiments, as an alternative to TET, the kit includes a chemical oxidative agent is included, e.g., potassium perruthenate (KRuO₄) or potassium ruthenate (K₂RuO₄).

In some embodiments, the kit further includes reagents for chemically blocking 5hmC from undergoing reactions that include 5mC. In some embodiments, the kit includes a glucose compound and a glucosyltransferase capable of transferring the glucose moiety to the 5-hydroxyl moiety of 5hmC. In some embodiments, the kit includes a beta-glucosyltransferase (BGT) and a UDP-glucose. In some embodiments, the BGT is T4 BGT.

In some embodiments, the method further comprises assessment of a status of a subject (*e.g.,* a patient) based on the methylation status of one or more genetic loci in the patient's genome. In some embodiments, the method comprises determining in the patient's sample, the genomic location and optionally, amount of methylated cytosines (5mC and/or 5hmC and/or 5fC and/or 5caC) in the genome. In some embodiments, genetic loci known to be biomarkers of disease are assessed for methylation. The method further comprises diagnosis of disease or condition in the patient or selecting or changing a treatment based on the presence or amount of methylation in the nucleic acid isolated from the patient.

Several methods exists for identifying disease or condition-specific methylation loci that can be assessed for methylation using the methods disclosed herein, *see e.g.,* U.S. Patent Publication No. US2020/0385813 "Systems and methods for estimating cell source fractions using methylation information;" US2020/0239965 "Source of origin deconvolution based on methylation fragments in cell-free DNA samples;" US2019/0287652 "Anomalous fragment detection and classification" (methylation markers indicating disease state); US2019/0316209 "Multi-assay prediction model for cancer detection;" US2019/0390257A1 "Tissue-specific methylation marker;" WO2011/070441 "Categorization of DNA samples;" WO2011/101728 "Identification of source of DNA samples;" WO2020/188561 "Methods and systems for detecting methylation changes in DNA samples."

In some embodiments, the invention includes a method of detecting tissue-specific DNA methylation patterns using the methylation detection methods disclosed herein. In one aspect of this embodiment, the method may further include identifying a tissue of origin of the methylated DNA present in the sample. In some embodiments, the method further includes identifying a tissue of origin of cell-free DNA isolated from blood. In another aspect of this embodiment, the invention includes detection of organ failure or organ injury, including organ transplant rejection in a transplant recipient using methylation patterns of cell-free DNA. The invention includes detecting circulating cell-free DNA with the organ-specific methylation pattern, wherein the presence of such cell-free DNA indicates organ transplant rejection. In some embodiments, the invention includes monitoring for transplant rejection by periodically sampling circulating cell-free DNA and measuring changes in the level of cell-free DNA with the organ-specific methylation pattern, wherein an increase in the level of such cell-free DNA indicates organ transplant rejection.

In some embodiments, the invention includes a method of diagnosis or screening for the presence of a cancerous tumor in a patient or subject. In some embodiments, the invention includes detection of a tumor using methylation patterns of cell-free DNA using the methylation detection methods disclosed herein. In some embodiments, the invention includes detecting a tumor originating from a particular tissue or organ by detecting circulating cell-free DNA with the tissue or organ-specific methylation pattern detected using the methylation detection methods disclosed herein, wherein the presence of such cell-free DNA indicates the presence of a tumor originating from the tissue or organ. In some embodiments, the invention includes monitoring the growth or shrinkage of a tumor by periodically sampling circulating cell-free DNA and measuring changes in the level of cell-free DNA with the tumor-specific methylation pattern, wherein an increase in the level of such cell-free DNA indicates tumor growth, while a decrease in the level of such cell-free DNA indicates tumor shrinkage.

In some embodiments, the invention includes a method of monitoring the effectiveness of treatment of cancer in a patient or subject. In some embodiments, the invention includes detection of tumor dynamics correlated with treatment using methylation patterns of cell-free DNA detected using the methylation detection methods disclosed herein. In some embodiments, the invention includes detecting effects of treatment on a tumor originating from a particular tissue or organ by periodically sampling circulating cell-free DNA and measuring changes in the level of cell-free DNA with the tissue or organ-specific methylation pattern, wherein an increase in the level of such cell-free DNA indicates tumor growth and ineffectiveness of treatment, while a decrease in the level of such cell-free DNA indicates tumor shrinkage and effectiveness of treatment, and a stable level of such cell-free DNA indicates stable disease and effectiveness of treatment.

In some embodiments, the invention includes a method of diagnosis or minimal residual disease (MRD) in a cancer patient following a treatment. National Cancer Institute defines MRD as a very small number of cancer cells that remain in the body during or after treatment when the patient has no signs or symptoms of the disease. In some embodiments, the invention includes a method of detecting MRD using methylation patterns of cell-free DNA detected using the methylation detection methods disclosed herein. In some embodiments, the invention includes detecting MRD from tumor originating from a particular tissue or organ by detecting circulating cell-free DNA with the tissue or organ-specific methylation pattern, wherein the presence of such cell-free DNA indicates the presence of MRD from the tumor.

In some embodiments, the invention includes a method of diagnosis or screening for the presence or status of an autoimmune disease in a patient or subject. In some embodiments, the invention includes detection of an autoimmune disease using methylation patterns of cell-free DNA detected using the methylation detection methods disclosed herein. In some embodiments, the invention includes detecting autoimmune disease characterized by damage to a particular tissue or organ by detecting circulating cell-free DNA with the tissue or organ-specific methylation pattern, wherein the presence of such cell-free DNA indicates organ damage resulting from the autoimmune disease and the presence of the autoimmune disease. In some embodiments, the invention includes monitoring for flare-ups or remission of an autoimmune disease by periodically sampling circulating cell-free DNA and measuring changes in the level of cell-free DNA with the tissue or organ-specific methylation pattern, wherein an increase in the level of such cell-free DNA indicates increased organ damage and a flare-up of the autoimmune disease, while a decrease in the level of such cell-free DNA indicates decreased organ damage and remission of the autoimmune disease.

## Claims

1. A method for reducing a C5-C6 double bond of cytosine in a nucleic acid, wherein the method comprises contacting cytosine in the nucleic acid with an ene reductase.

2. The method of claim 1, wherein the cytosine is selected from a group consisting of 5-Formylcytosine (5fC), 5-Carboxylcytosine (5caC), 5,6-dihydro-fC, 5,6-dihydro-caC, 2'-Deoxy-5-formylcytidine, 2'-Deoxy-5-carboxycytidine, 5-Formyl-2'-deoxycytidine, 5-Formyl-dC, 5-Carboxy-2'-deoxycytidine, and 5-Carboxy-dC,

3. The method of claim 1, wherein contacting the cytosine in the nucleic acid with an ene reductase reduces the cytosine to 5,6-dihydro-U (DHU).

4. The method of claim 3, wherein the DHU is subsequently converted to a Thymine.

5. The method of claim 1, wherein reduction of the C5-C6 double bond of cytosine in a nucleic acid by contacting the cytosine in the nucleic acid with an ene reductase is a part of a method for detecting an epigenetic cytosine modification.

6. A method for detecting an epigenetic cytosine modification, wherein the method comprises at least the step of reducing a C5-C6 double bond of cytosine in a nucleic acid by contacting cytosine in the nucleic acid with an ene reductase.

7. The method of claim 6, wherein the cytosine is selected from a group consisting of 5-Formylcytosine (5fC), 5-Carboxylcytosine (5caC), 5,6-dihydro-fC, 5,6-dihydro-caC, 2'-Deoxy-5-formylcytidine, 2'-Deoxy-5-carboxycytidine, 5-Formyl-2'-deoxycytidine, 5-Formyl-dC, 5-Carboxy-2'-deoxycytidine and 5-Carboxy-dC.

8. The method of claim 6, wherein contacting the cytosine in the nucleic acid with an ene reductase reduces the cytosine to 5,6-dihydro-U (DHU).

9. The method of claim 8, wherein the DHU is subsequently converted to a thymine.

## Patentansprüche

1. Verfahren zur Reduktion einer C5-C6-Doppelbindung von Cytosin in einer Nukleinsäure, wobei das Verfahren das Inkontaktbringen von Cytosin in der Nukleinsäure mit einer En-Reduktase umfasst.

2. Verfahren nach Anspruch 1, wobei das Cytosin ausgewählt ist aus einer Gruppe, bestehend aus 5-Formylcytosin (5fC), 5-Carboxylcytosin (5caC), 5,6-Dihydro-fC, 5,6-Dihydro-caC, 2'-Desoxy-5-formylcytidin, 2'-Desoxy-5-carboxycytidin, 5-Formyl-2'-desoxycytidin, 5-Formyl-dC, 5-Carboxy-2'-desoxycytidin und 5-Carboxy-dC.

3. Verfahren nach Anspruch 1, wobei das Inkontaktbringen des Cytosins in der Nukleinsäure mit einer En-Reduktase das Cytosin zu 5,6-Dihydro-U (DHU) reduziert.

4. Verfahren nach Anspruch 3, wobei das DHU anschließend in ein Thymin umgewandelt wird.

5. Verfahren nach Anspruch 1, wobei die Reduktion der C5-C6-Doppelbindung von Cytosin in einer Nukleinsäure durch Inkontaktbringen des Cytosins in der Nukleinsäure mit einer En-Reduktase Teil eines Verfahrens zum Nachweis einer epigenetischen Cytosinmodifikation ist.

6. Verfahren zum Nachweis einer epigenetischen Cytosinmodifikation, wobei das Verfahren mindestens den Schritt der Reduktion einer C5-C6-Doppelbindung von Cytosin in einer Nukleinsäure durch Inkontaktbringen von Cytosin in der Nukleinsäure mit einer En-Reduktase umfasst.

7. Verfahren nach Anspruch 6, wobei das Cytosin ausgewählt ist aus einer Gruppe, bestehend aus 5-Formylcytosin (5fC), 5-Carboxylcytosin (5caC), 5,6-Dihydro-fC, 5,6-Dihydro-caC, 2'-Desoxy-5-formylcytidin, 2'-Desoxy-5-carboxycytidin, 5-Formyl-2'-desoxycytidin, 5-Formyl-dC, 5-Carboxy-2'-desoxycytidin und 5-Carboxy-dC.

8. Verfahren nach Anspruch 6, wobei das Inkontaktbringen des Cytosins in der Nukleinsäure mit einer En-Reduktase das Cytosin zu 5,6-Dihydro-U (DHU) reduziert.

9. Verfahren nach Anspruch 8, wobei das DHU anschließend in ein Thymin umgewandelt wird.

## Revendications

1. Procédé de réduction d'une double liaison C5-C6 de cytosine dans un acide nucléique, dans lequel le procédé comprend la mise en contact de la cytosine dans l'acide nucléique avec une ène réductase.

2. Procédé selon la revendication 1, dans lequel la cytosine est choisie dans un groupe constitué par la 5-formylcytosine (5fC), la 5-carboxylcytosine (5caC), la 5,6-dihydro-fC, la 5,6-dihydro-caC, la 2'-désoxy-5-formylcytidine, la 2'-désoxy-5-carboxycytidine, la 5-formyl-2'-désoxycytidine, la 5-formyl-dC, la 5-carboxy-2'-désoxycytidine et la 5-carboxy-dC.

3. Procédé selon la revendication 1, dans lequel la mise en contact de la cytosine dans l'acide nucléique avec une ène réductase réduit la cytosine en 5,6-dihydro-U (DHU).

4. Procédé selon la revendication 3, dans lequel le DHU est ensuite converti en une thymine.

5. Procédé selon la revendication 1, dans lequel la réduction de la double liaison C5-C6 de la cytosine dans un acide nucléique en mettant en contact la cytosine dans l'acide nucléique avec une ène réductase est une partie d'un procédé de détection d'une modification épigénétique de cytosine.

6. Procédé de détection d'une modification épigénétique de cytosine, dans lequel le procédé comprend au moins l'étape de réduction d'une double liaison C5-C6 de la cytosine dans un acide nucléique en mettant en contact la cytosine dans l'acide nucléique avec une ène réductase.

7. Procédé selon la revendication 6, dans lequel la cytosine est choisie dans un groupe constitué par la 5-formylcytosine (5fC), la 5-carboxylcytosine (5caC), la 5,6-dihydro-fC, la 5,6-dihydro-caC, la 2'-désoxy-5-formylcytidine, la 2'-désoxy-5-carboxycytidine, la 5-formyl-2'-désoxycytidine, la 5-formyl-dC, la 5-carboxy-2'-désoxycytidine et la 5-carboxy-dC.

8. Procédé selon la revendication 6, dans lequel la mise en contact de la cytosine dans l'acide nucléique avec une ène réductase réduit la cytosine en 5,6-dihydro-U (DHU).

9. Procédé selon la revendication 8, dans lequel le DHU est ensuite converti en une thymine.
